# EUROPEAN PATENT APPLICATION

(11) **EP 4 289 448 A1**
(43) Date of publication of application: **13.12.2023**
(21) Application number: 22196781.3
(22) Date of filing: 21.09.2022
(51) Int. Cl.: A61L 2/20, A61L 2/24, A61L 9/015, A61L 9/03, B60H 3/00

(54) **STERILIZATION APPARATUS FOR VEHICLE AND STERILIZATION SYSTEM FOR VEHICLE INCLUDING THE SAME**

(30) Priority: 08.06.2022 KR 20220069497
(71) Applicant: Hyundai Mobis Co., Ltd., Gangnam-gu Seoul 06141 (KR)
(72) Inventor: KIM, Seung Cheol, 16891 Gyeonggi-do (KR)
(74) Representative: Vossius & Partner Patentanwälte Rechtsanwälte mbB

(57) **Abstract**

The present disclosure relates to a sterilization apparatus for a vehicle and a sterilization system for a vehicle including the same. The sterilization apparatus for a vehicle includes a housing in which a connection part is formed to be connected to a vehicle vent, a sterilization unit disposed inside the housing, and configured to generate a sterilization material to discharge the sterilization material toward the vent, and a drive source configured to drive the sterilization unit so that the sterilization unit communicates with the connection part to enable the sterilization material to be discharged.

## Description

### CROSS-REFERENCE TO RELATED APPLICATION

This application claims priority to and the benefit of Korean Patent Application No. 10-2022-0069497, filed on June 08, 2022, the disclosure of which is incorporated herein by reference in its entirety.

### BACKGROUND

### 1. Field of the Disclosure

The present disclosure relates to a sterilization apparatus for a vehicle and a sterilization system for a vehicle including the same.

### 2. Discussion of Related Art

In general, since a large number of people may ride in a vehicle, secretions such as sweat and saliva of passengers or foreign substances stained on clothes and bags of the passengers may remain inside the vehicle.

As described above, when the secretions or foreign substances remaining inside the vehicle come into contact with the passengers' bodies, it may cause skin diseases, and when the secretions or foreign substances enter the inside of the body through the mouth, it may cause bronchial disease, such as bronchitis.

Recently, as interest in improving hygiene in a passenger compartment increases, a sterilization apparatus for a vehicle for improving the cleanliness of the passenger compartment has been developed. A conventional sterilization apparatus for a vehicle may perform a sterilization operation while the vehicle travels.

However, since the conventional sterilization apparatus for a vehicle performs the sterilization operation while the vehicle travels, it is harmless to the human body and is required to maintain a sterilization level that does not interfere with the traveling, so that there is a problem in that the sterilization effect is insignificant.

Accordingly, there is a need for research to improve the cleanliness of the passenger compartment by performing a sterilization operation having a strong sterilization level.

### SUMMARY OF THE DISCLOSURE

The present disclosure is directed to providing a sterilization apparatus for a vehicle and a sterilization system for a vehicle including the same, which are improved so that a sterilization operation having a strong sterilization level may be performed inside a passenger compartment after passengers exit the vehicle.

One embodiment of the present disclosure provides a sterilization apparatus for a vehicle including: a housing in which a connection part is formed to be connected to a vehicle vent; a sterilization unit disposed inside the housing, and configured to generate a sterilization material to discharge the sterilization material toward the vent; and a drive source configured to drive the sterilization unit so that the sterilization unit communicates with the connection part to enable the sterilization material to be discharged.

The sterilization unit may include: a first sterilization part configured to generate ozone; and a second sterilization part configured to store a sterilization solution inside the housing and vaporize the sterilization solution.

The sterilization unit may include a rotation part configured to rotate the first sterilization part and the second sterilization part, and the rotation part may include: a rotator configured to form a plurality of chambers in which the first sterilization part and the second sterilization part are disposed; and a rotation shaft connected to the rotator and the drive source to rotate the rotator through power generated from the drive source.

When the rotator is rotated, the first sterilization part and the second sterilization part may be disposed to communicate with the connection part of the housing.

The second sterilization part may include: a storage part configured to store the sterilization solution; and a vaporization part configured to generate mist by vaporizing the sterilization solution stored in the storage part.

The plurality of chambers may include: a first chamber in which the first sterilization part is disposed; a second chamber in which the storage part is disposed; a third chamber in which the vaporization part is disposed; and a fourth chamber having a closed shape so as not to communicate with the connection part of the housing in an air-conditioning operating state of an air-conditioning apparatus.

The vaporization part may include: a pump configured to pump the sterilization solution stored in the storage part; a nozzle connected to the pump to guide the pumped sterilization solution; a heater wound around the nozzle, and configured to heat the sterilization solution that moves inside the nozzle; and a heat transfer body configured to accommodate a part of the nozzle and the heater therein, and transmit heat generated from the heater to the nozzle.

The sterilization unit may include a chamber cover configured to cover an open one side of the third chamber, the chamber cover may be includes a first exposure hole through which an end of the nozzle passes, and the heat transfer body may be formed with a second exposure hole configured to communicate with the first exposure hole, and through which the end of the nozzle passes together with the first exposure hole.

The rotator may be formed with a first inflow hole formed in an area corresponding to the second chamber, the storage part may be formed with a second inflow hole communicating with the first inflow hole, and the housing may include an inflow pipe configured to communicate with the first inflow hole and the second inflow hole, and guide the sterilization solution to an inside of the storage part.

The sterilization apparatus may include a controller electrically connected to the drive source to transmit a rotation signal to the drive source, wherein the sterilization unit is rotated by the drive source to form a movement path of the sterilization material together with the connection part and the vent.

### BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
FIG. 1 is a perspective view of a sterilization apparatus for a vehicle according to one embodiment of the present disclosure;
FIG. 2 is a bottom perspective view of the sterilization apparatus for a vehicle according to one embodiment of the present disclosure;
FIG. 3 is a perspective view of a housing;
FIG. 4 is a cross-sectional view taken along line A-A in FIG. 3;
FIG. 5 is a perspective view of a sterilization unit;
FIG. 6 is a cross-sectional view showing a state in which the sterilization unit is accommodated inside a first accommodation part of the housing;
FIG. 7 is a perspective view of a rotator;
FIG. 8 is a view showing a state in which a fourth chamber and a second connection part are disposed to face each other;
FIG. 9 is a view showing a state in which a holder is separated from a rotary shaft;
FIG. 10 is a view showing a state in which a chamber cover and a side cover are coupled to the rotator;
FIG. 11 is a cross-sectional view of the rotator, the chamber cover, and the side cover;
FIG. 12 is a view showing a state in which a first sterilization part is disposed in a first chamber;
FIG. 13 is a view showing a state in which the first sterilization part and a second connection part are disposed to face each other;
FIG. 14 is a view showing a state in which a storage part is separated from a second chamber;
FIG. 15 is a view showing a state in which a vaporization part is disposed in a third chamber;
FIG. 16 is a view showing a state in which a heat transfer body has been removed from the vaporization part;
FIG. 17 is a view showing a state in which the vaporization part is disposed to face the second connection part;
FIG. 18 is a view showing a state in which a drive source is coupled to the rotary shaft;
FIG. 19 is a view showing a state in which a controller case has been removed from a housing;
FIG. 20 is a view showing a cable connected to a pump and a controller;
FIG. 21 is a view showing a cable connected to the first sterilization part and the controller and a cable connected to a heater and the controller;
FIG. 22 is a view showing a state in which a side cover covers an open one side of a concave portion;
FIG. 23 is a view schematically showing a sterilization system for a vehicle according to one embodiment of the present disclosure;
FIG. 24 is a view showing a configuration of a display;
FIG. 25 is a view showing an operation order of a first sterilization mode;
FIG. 26 is a view showing an operation order of a first sterilization end mode;
FIG. 27 is a view showing an operation order of a second sterilization mode;
FIG. 28 is a view showing an operation order of a second sterilization end mode; and
FIG. 29 is a view showing an operation order of a continuous sterilization mode.

### DETAILED DESCRIPTION OF EXEMPLARY EMBODIMENTS

Since the present disclosure may have various changes and have various embodiments, specific embodiments are illustrated and described in the drawings. However, it should be understood that this is not intended to limit the present disclosure to specific embodiments, and includes all modifications, equivalents, and substitutes included in the spirit and technical scope of the present disclosure.

Terms including an ordinal number, such as first and second, may be used to describe various components, but the components are not limited by the terms. The above terms are used only for the purpose of distinguishing one component from another. For example, a second component may be referred to as a first component, and similarly, the first component may also be referred to as the second component without departing from the scope of the present disclosure. The term "and/or" includes a combination of a plurality of related listed items or any of the plurality of related listed items.

It should be understood that when it is mentioned that a component is "connected" or "joined" to another component, it may be directly connected or joined to another component, but other components may also exist therebetween. On the other hand, it should be understood that when it is mentioned that a component is "directly connected" or "directly joined" to another component, no other components exists therebetween.

In the description of the embodiments, when it is described that any one component is formed "on or under" another component, the "on or under" includes both a case in which two components come into direct contact with each other or a case in which one or more other components are disposed between the two components. In addition, when expressed as "on or under", it may also include the meaning of not only upward but also downward with respect to one component.

The terms used in this application are only used to describe specific embodiments and are not intended to limit the present disclosure. The singular expression includes the plural expression unless the context clearly dictates otherwise. In this application, it should be understood that terms such as "comprise" or "have" are intended to designate that a feature, number, step, operation, component, part, or combination thereof described in the specification exists, and do not preclude the possibility of presence or addition of one or more other features, numbers, steps, operations, components, parts, or combinations thereof in advance.

Unless defined otherwise, all terms used herein, including technical or scientific terms, have the same meaning as commonly understood by those skilled in the art to which the present disclosure pertains. Terms such as those defined in a commonly used dictionary should be interpreted as having a meaning consistent with the meaning in the context of the related art, and should not be interpreted in an ideal or excessively formal meaning unless explicitly defined in this application.

Hereinafter, a sterilization apparatus for a vehicle will be described in detail with reference to the accompanying drawings, and the same or corresponding components are given the same reference numbers regardless of reference numerals, and overlapping descriptions thereof will be omitted.

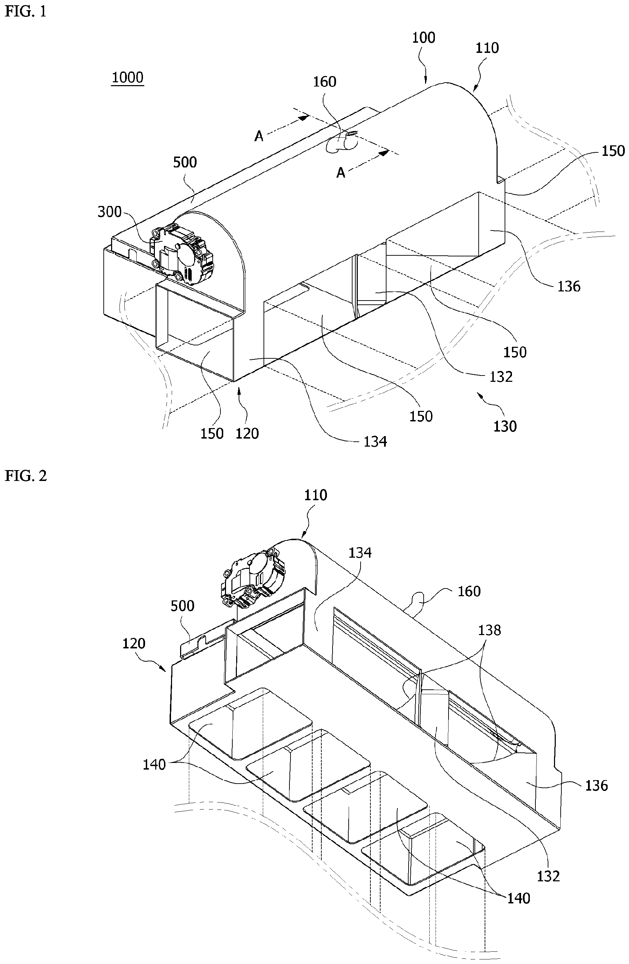

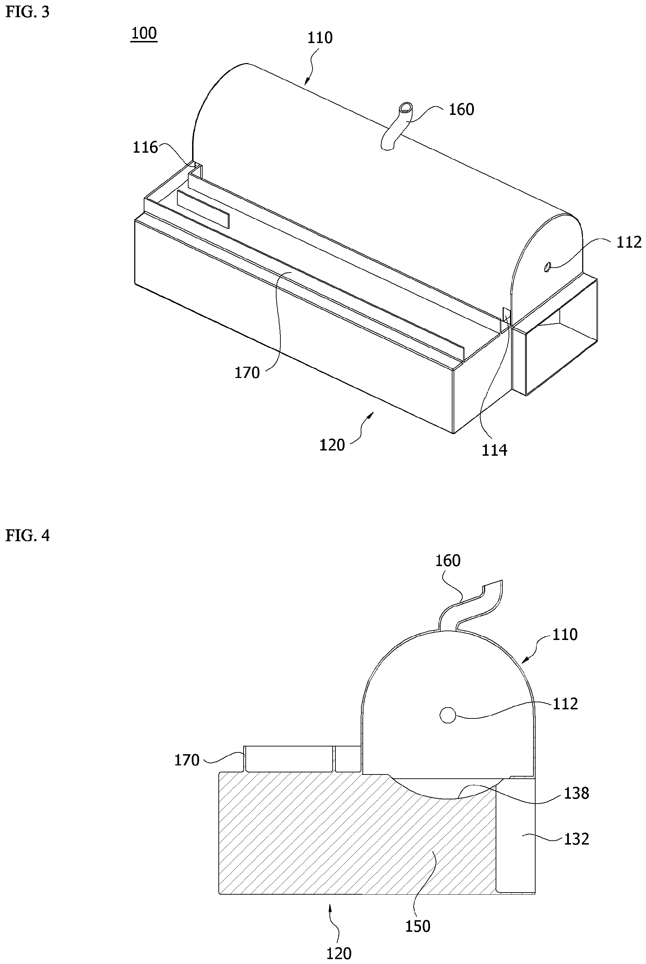

FIG. 1 is a perspective view of a sterilization apparatus for a vehicle according to one embodiment of the present disclosure, FIG. 2 is a bottom perspective view of the sterilization apparatus for a vehicle according to one embodiment of the present disclosure, FIG. 3 is a perspective view of a housing, and FIG. 4 is a cross-sectional view taken along line A-A in FIG. 3.

Referring to FIGS. 1 to 4, a sterilization apparatus 1000 for a vehicle according to one embodiment of the present disclosure may include a housing 100 in which connection parts 140 and 150 are formed to be connected to a vehicle vent, a sterilization unit 200 disposed inside the housing 100 and configured to discharge a sterilization material toward the vehicle vent by generating the sterilization material, a drive source 300 configured to enable the sterilization material to be discharged by driving the sterilization unit 200 so that the sterilization unit 200 communicates with the connection parts 140 and 150, a controller 400 (e.g., processor, CPU, etc.) electrically connected to the drive source 300 to transmit a rotation signal to the drive source 300, and a controller case 500 configured to protect the controller 400.

The housing 100 may include a first accommodation part 110 configured to accommodate the sterilization unit 200, a second accommodation part 120 connected to the vehicle vent, a plurality of partition frames 130 configured to partition an inside of the second accommodation part 120, a first connection part 140 connected to a discharge part of an air-conditioning apparatus (not shown) to introduce air moving from the air-conditioning apparatus, a second connection part 150 connected to a vehicle air vent (not shown) so that the air moved into the housing 100 is discharged to the vehicle air vent, an inflow pipe 160 formed to replenish a sterilization solution, and a holding frame 170 configured to hold the controller 400.

The first accommodation part 110 may have a hollow shape so that the sterilization unit 200 may be disposed therein. A part (e.g., a top) of the first accommodation part 110 may be formed in a curved shape so as not to interfere with the rotation of a rotator 212 to be described below.

The first accommodation part 110 may include a support hole 112, a first through hole 114, and a second through hole 116.

The support hole 112 may be disposed on each of the sides of the first accommodation part 110. The support hole 112 may be connected to the inside and outside of the first accommodation part 110 so that a part of a rotation shaft 214 of a rotation part 210 to be described below passes therethrough. The support hole 112 may be provided in a shape corresponding to a shape of the rotation shaft 214. Accordingly, the support hole 112 may support a part of the rotation shaft 214.

The first through hole 114 may be disposed in a part of the first accommodation part 110 that meets the second accommodation part 120. The first through hole 114 may allow a part of a cable 420 connected to the controller 400 to pass through the inside of the first accommodation part 110 or a part of the cable 420 disposed inside the first accommodation part 110 to pass through the outside of the first accommodation part 110.

The second through hole 116 may be disposed in a part of the first accommodation part 110 that meets the second accommodation part 120. The second through hole 116 may be disposed to be spaced apart from the first through hole 114. The second through hole 116 may allow the part of the cable 420 connected to the controller 400 to pass through the inside of the first accommodation part 110 or the part of the cable 420 disposed inside the first accommodation part 110 to pass through the outside of the first accommodation part 110.

The second accommodation part 120 may be connected to an end of the first accommodation part 110. The second accommodation part 120 may be provided in a hollow shape to form a movement path of air in a state of being connected to the first accommodation part 110. The second accommodation part 120 may protrude further than the first accommodation part 110 in a direction crossing a longitudinal direction of the first accommodation part 110. The holding frame 170, the controller 400, the cable 420, and the controller case 500 may be disposed in a portion of the second accommodation part 120 that protrudes further than the first accommodation part 110.

The plurality of partition frames 130 may be disposed inside the second accommodation part 120. The plurality of partition frames 130 may partition the second accommodation part 120 so that a plurality of areas are formed inside the second accommodation part 120. Accordingly, the plurality of partition frames 130 may include a first partition frame 132, a second partition frame 134, a third partition frame 136, and a support groove 138 so that the inside of the second accommodation part 120 may be used as the movement passage of the air connected to the vehicle vent through the plurality of partition frames 130.

The first partition frame 132 may be disposed at a central portion of the inside of the second accommodation part 120. The inside of the second accommodation part 120 may be divided into two areas by the first partition frame 132.

The second partition frame 134 may be disposed in any one of the two areas partitioned by the first partition frame 132. The third partition frame 136 may be disposed in the other one of the two areas partitioned by the first partition frame 132. Four areas may be formed inside the second accommodation part 120 by the second partition frame 134 and the third partition frame 136.

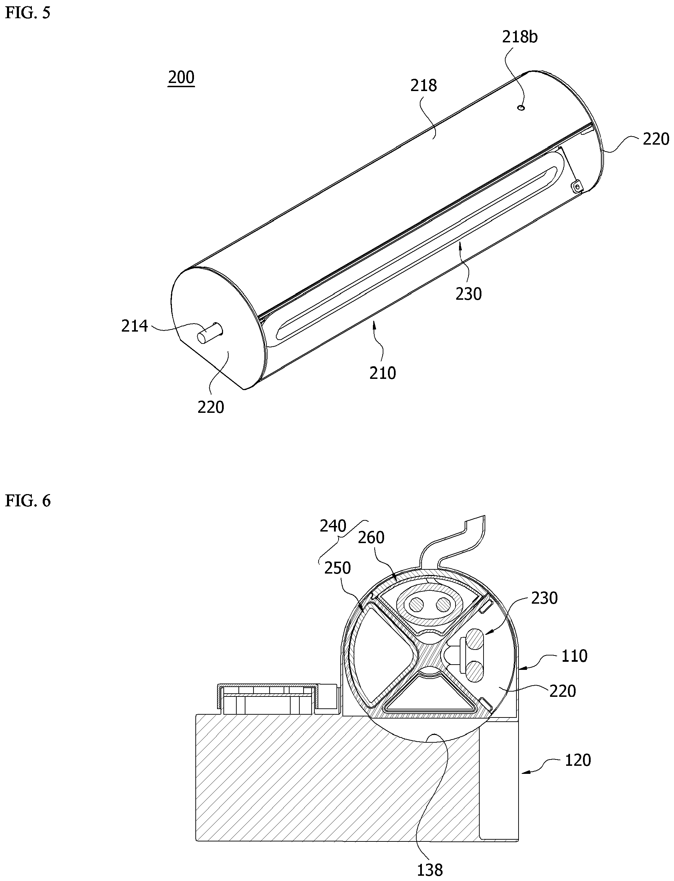

The support groove 138 may be formed at an end of each of the first partition frame 132, the second partition frame 134, and the third partition frame 136. As shown in FIG. 6, the support groove 138 may support both sides of the rotator 212 to be described below disposed in the first accommodation part 110.

The support groove 138 may be formed as a concentric circle with the support hole 112. In addition, the support groove 138 may have a shape corresponding to an outer surface of the rotator 212. Accordingly, the support groove 138 may not interfere with the rotation of the rotator 212 rotated around the rotation shaft 214 supported by the support hole 112.

The first connection part 140 may be disposed on one surface (e.g., a bottom) of the second accommodation part 120. More specifically, the first connection part 140 may be provided in a hole shape to be connected to the plurality of areas partitioned by the plurality of partition frames 130. The first connection part 140 may be connected to an exhaust duct of the air-conditioning apparatus for a vehicle. The first connection part 140 may be used as a passage in which air moving from the air conditioning apparatus for a vehicle may be introduced into the second accommodation part 120.

The second connection part 150 may be formed inside the second accommodation part 120 and on surfaces of the second accommodation part 120. More specifically, the second connection part 150 may be composed of a combination of four areas formed by the plurality of partition frames 130 and holes formed on each surface except for the bottom of the second accommodation part 120. The second connection part 150 may be used as the movement passage of the air so that the air introduced from the exhaust duct of the air-conditioning apparatus for a vehicle connected to the first connection part 140 may be mixed with the sterilization material generated from the sterilization unit 200 and moved to the inside of the vehicle. A duct of the vehicle air vent may be connected to the second connection part 150. Accordingly, the air introduced into the second accommodation part 120 may be discharged into the vehicle through the inflow duct of the vehicle air vent connected to the second connection part 150.

The inflow pipe 160 may be disposed on an outer surface of the first accommodation part 110. The inflow pipe 160 may communicate with a first inflow hole 213a and a second inflow hole 252, which will be described below. The inflow pipe 160 may guide the sterilization solution to an inside of the storage part 250 so that the sterilization solution may be introduced into the storage part 250 to be described below. Although not shown, the inflow pipe 160 may be connected to a crash pad of the vehicle, and an inlet of the inflow pipe 160 may be closed by a cap installed on the crash pad.

The holding frame 170 may be disposed on an outer surface of the second accommodation part 120. More specifically, as shown in FIG. 3, the holding frame 170 may be disposed on an outer surface of a portion of the second accommodation part 120 protruding in a direction crossing the longitudinal direction of the first accommodation part 110. The holding frame 170 may protrude from the second accommodation part 120 toward the first accommodation part 110. The holding frame 170 can prevent the controller 400 from being separated by accommodating the controller 400 therein.

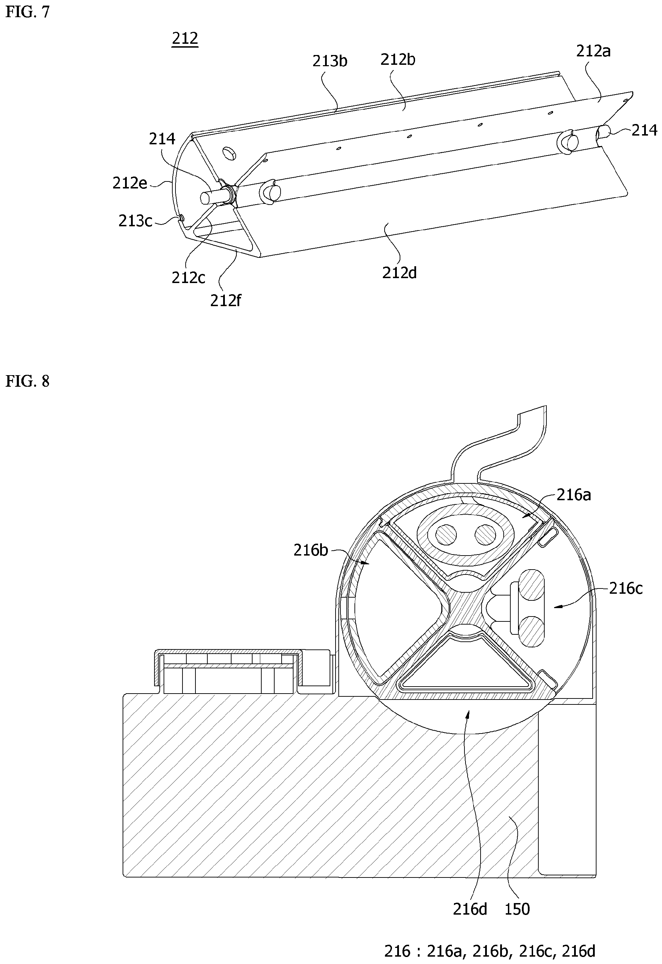

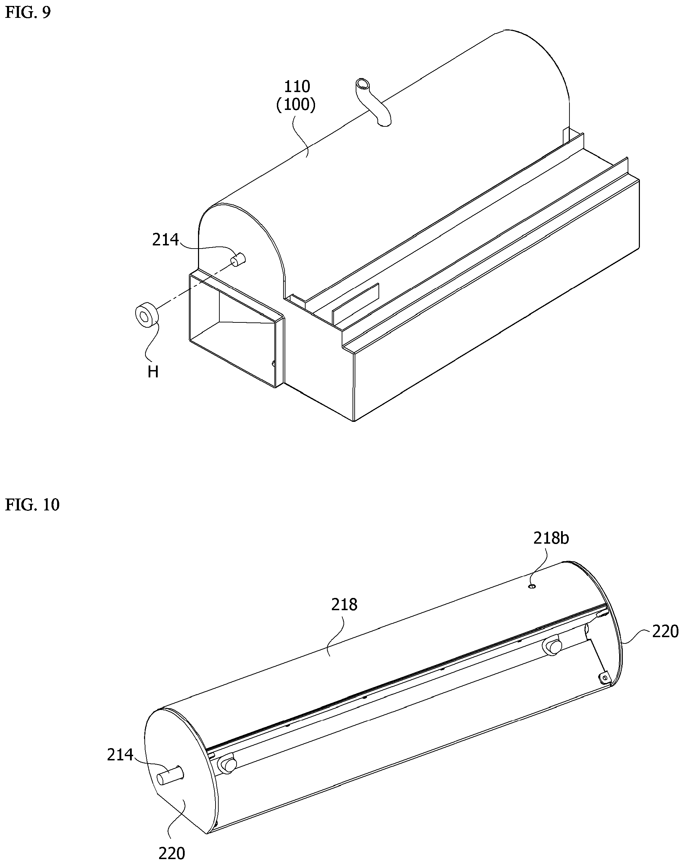

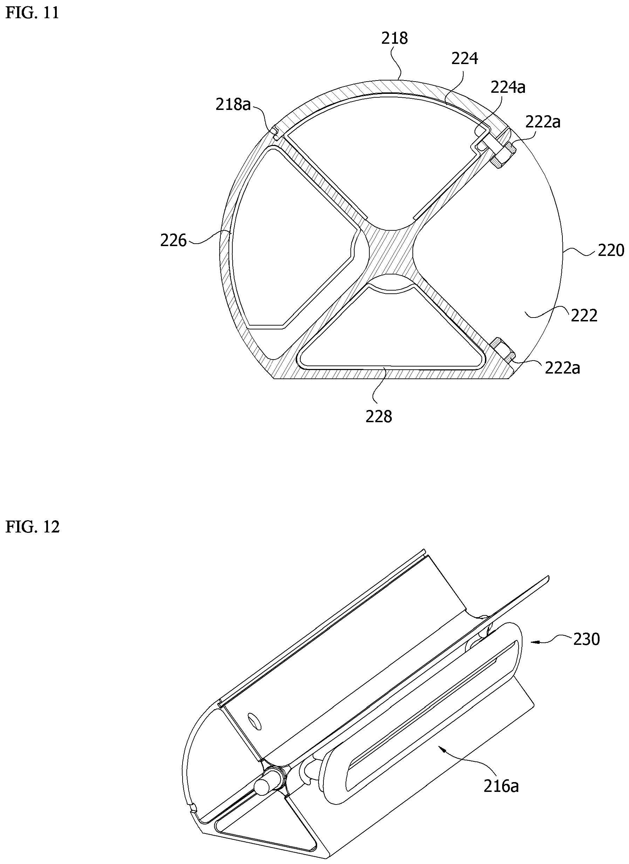

FIG. 5 is a perspective view of a sterilization unit, FIG. 6 is a cross-sectional view showing a state in which the sterilization unit is accommodated inside a first accommodation part of the housing, FIG. 7 is a perspective view of a rotator, FIG. 8 is a view showing a state in which a fourth chamber and a second connection part are disposed to face each other, FIG. 9 is a view showing a state in which a holder is separated from a rotary shaft, FIG. 10 is a view showing a state in which a chamber cover and a side cover are coupled to the rotator, and FIG. 11 is a cross-sectional view of the rotator, the chamber cover, and the side cover.

Referring to FIGS. 5 to 11, the sterilization unit 200 may be disposed inside the first accommodation part 110. The sterilization unit 200 may generate the sterilization material according to a signal of the controller 400 in a state of being disposed inside the first accommodation part 110. In addition, the sterilization unit 200 may be rotated by the drive source 300 to form the movement path of the sterilization material together with the second connection part 150 and the air vent.

The sterilization unit 200 may include the rotation part 210, a first sterilization part 230, and a second sterilization part 240.

The rotation part 210 may rotate the first sterilization part 230 and the second sterilization part 240. The rotation part 210 may be rotated based on power generated from the drive source 300.

The rotation part 210 may include the rotator 212, the rotation shaft 214, a plurality of chambers 216, a chamber cover 218, and a side cover 220.

The rotator 212 may form the plurality of chambers 216 in which the first sterilization part 230 and the second sterilization part 240 are disposed. The rotator 212 may include a first partition 212a, a second partition 212b, a third partition 212c, a fourth partition 212d, a first cover frame 212e, and a second cover frame 212f.

As shown in FIGS. 7 and 11, the first partition 212a, the second partition 212b, the third partition 212c, and the fourth partition 212d may have an X shape that is a shape in which each end meets at one point. The first partition 212a may form a straight wall together with the third partition 212c, and the second partition 212b may form a straight wall together with the fourth partition 212d. The first partition 212a to the fourth partition 212d may be provided to have lengths corresponding to each other. Accordingly, when the rotator 212 is rotated, the first cover frame 212e and the chamber cover 218 may be rotated stably without colliding with the support grooves 138 of the plurality of partition frames 130.

The first cover frame 212e may be coupled to each of the second partition 212b and the third partition 212c. The first cover frame 212e may form a sectoral shape together with the second partition 212b and the third partition 212c. The first cover frame 212e may have a shape corresponding to a curved inner surface shape of the first accommodation part 110. Accordingly, when the rotator 212 rotates, the first cover frame 212e may be smoothly rotated inside the first accommodation part 110 of the housing 100.

The second cover frame 212f may be coupled to each of the third partition 212c and the fourth partition 212d. The second cover frame 212f may form a triangular shape together with the third partition 212c and the fourth partition 212d. The second cover frame 212f may be provided in a non-curved straight shape. The second cover frame 212f may form a closed circuit with the third partition 212c and the fourth partition 212d. Accordingly, when the second cover frame 212f is disposed to face the second connection part 150, the second cover frame 212f may block the air introduced into the second accommodation part 120 from being introduced into the first accommodation part 110.

The rotator 212 may further include the first inflow hole 213a, a coupling groove 213b, a first side groove 213c, and a second side groove 213d.

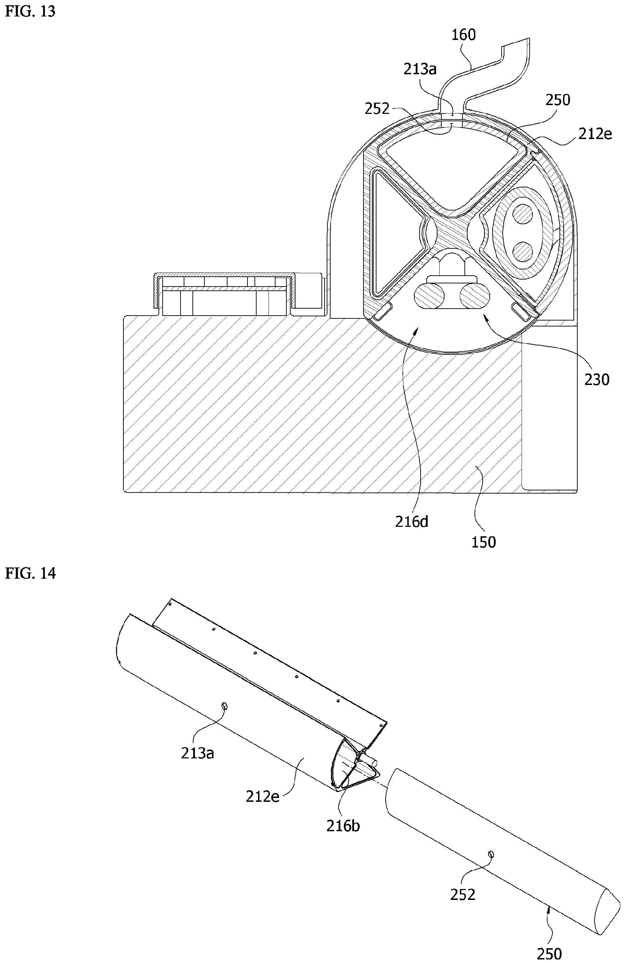

The first inflow hole 213a may be formed in an area corresponding to a second chamber 216b to be described below of the rotation part 210. More specifically, as shown in FIG. 14, the first inflow hole 213a may be disposed at the central portion of the first cover frame 212e, but is not limited thereto. When the rotator 212 is rotated and the first cover frame 212e is disposed at one side (e.g., a top) of the first accommodation part 110, the first inflow hole 213a may communicate with the inflow pipe 160 of the housing 100. The first inflow hole 213a may be used as a passage in which the sterilization solution introduced through the inflow pipe 160 moves to the inside of the storage part 250.

The coupling groove 213b may be disposed in the second partition 212b. The coupling groove 213b may be formed in the longitudinal direction of the second partition 212b. The coupling groove 213b may be concavely formed inside the second partition 212b so that a part of the chamber cover 218 is slidably movable. The coupling groove 213b may be disposed at an outside of the first support frame 224 of the side cover 220 to be described below.

The first side groove 213c may be disposed at one end of the first cover frame 212e. The first side groove 213c may be used as the passage through which the cable 420 to be described below passes. When the side cover 220 is coupled to the rotator 212, an open one side of the first side groove 213c may be covered. The cable 420 disposed in the first side groove 213c may not be separated from the outside of the first side groove 213c by the side cover 220.

The second side groove 213d may be disposed at the other end of the first cover frame 212e. The second side groove 213d may be used as the passage through which the cable 420 to be described below passes. When the side cover 220 is coupled to the rotator 212, an open one side of the second side groove 213d may be covered. The cable 420 disposed in the second side groove 213d may not be separated from the outside of the second side groove 213d by the side cover 220.

The rotation shaft 214 may be disposed at both sides of the first partition 212a to the fourth partition 212d. The rotation shaft 214 may be provided in a shape protruding in the longitudinal directions of the first partition 212a to the fourth partition 212d. The rotation shaft 214 may be supported by the support hole 112 of the first accommodation part 110 of the housing 100. A part of the rotation shaft 214 may be exposed to the outside of the housing 100 in a state of being supported by the support hole 112. The rotation shaft 214 may be connected to the rotator 212 and the drive source 300 to rotate the rotator 212 through power generated from the drive source 300.

In addition, a holder H may be coupled to the other one of the rotation shafts 214 disposed at both sides of the first partition 212a to the fourth partition 212d that is not connected to the drive source 300. As shown in FIG. 9, the holder H can be coupled to the rotation shaft 214 exposed to the outside of the first accommodation part 110 of the housing 100 to prevent the rotation shaft 214 from being separated to the inside of the first accommodation part of the housing 100. An inner surface of the holder H may be provided in a shape corresponding to an outer surface of the rotation shaft 214.

The plurality of chambers 216 may be formed by the first partition 212a to the fourth partition 212d. The plurality of chambers 216 may include a first chamber 216a, a second chamber 216b, a third chamber 216c, and a fourth chamber 216d.

The first chamber 216a may be formed by the first partition 212a and the fourth partition 212d. The first sterilization part 230 may be disposed in the first chamber 216a. An opening having an open one side may be formed in the first chamber 216a. Accordingly, the sterilization material generated from the first sterilization part 230 disposed in the first chamber 216a may move from the first chamber 216a to the second connection part 150 by the opening of the first chamber 216a.

The second chamber 216b may be formed by the second partition 212b, the third partition 212c, and the first cover frame 212e. A sectoral-shaped inner space may be formed in the second chamber 216b by the second partition 212b, the third partition 212c, and the first cover frame 212e. The second chamber 216b may have an inner space symmetrical to the first chamber 216a, but is not limited thereto. The storage part 250 to be described below of the second sterilization part 240 may be disposed inside the second chamber 216b.

The third chamber 216c may be formed by the first partition 212a, the second partition 212b, and the chamber cover 218. A sectoral-shaped inner space may be formed in the third chamber 216c by the first partition 212a, the second partition 212b, and the chamber cover 218. A vaporization part 260 of the second sterilization part 240 may be disposed inside the third chamber 216c.

The fourth chamber 216d may be formed by the third partition 212c, the fourth partition 212d, and the second cover frame 212f. A triangular-shaped inner space may be formed in the fourth chamber 216d by the third partition 212c, the fourth partition 212d, and the second cover frame 212f. The fourth chamber 216d may have a closed shape so that the first chamber 216a and the third chamber 216c do not communicate with the second connection part 150 of the housing 100 in an air-conditioning operating state of the air-conditioning apparatus. Accordingly, as shown in FIG. 8, when the fourth chamber 216d is disposed to face the second connection part 150 of the housing 100, the air introduced into the second accommodation part 120 of the housing 100 can be prevented from being introduced into the first accommodation part 110 by the fourth chamber 216d in the air-conditioning operating state of the air-conditioning apparatus.

The first chamber 216a to the fourth chamber 216d may be rotated by the rotation of the rotation shaft 214. More specifically, when the rotation shaft 214 is rotated by the driving force generated from the drive source 300, the first chamber 216a to the fourth chamber 216d may communicate with the second connection part 150 of the housing 100. Accordingly, the sterilization material generated from the vaporization part 260 to be described below of the first sterilization part 230 disposed in the first chamber 216a and the second sterilization part 240 disposed in the third chamber 216c may move from the second connection part 150 to the vehicle air vent together with the air to move to a vehicle passenger compartment.

The chamber cover 218 may cover an open one side of the third chamber 216c. More specifically, the chamber cover 218 may be disposed at an open one side formed through the first partition 212a and the second partition 212b to seal a space formed through the first partition 212a and the second partition 212b.

The chamber cover 218 may be formed to have a curvature corresponding to a curvature of the first cover frame 212e. In addition, the chamber cover 218 may have a shape corresponding to the curved shape of the first accommodation part 110 of the housing 100. Accordingly, when the rotator 212 is rotated, the chamber cover 218 may be rotated by the rotation shaft 214 without colliding with the inner surface of the first accommodation part 110. The chamber cover 218 can be prevented from being shaken left or right in the longitudinal direction of the rotator 212 by the side cover 220.

The chamber cover 218 may include a first coupling protrusion 218a and a first exposure hole 218b.

The first coupling protrusion 218a may be disposed at one end of the chamber cover 218. More specifically, when the chamber cover 218 is coupled to the first partition 212a and the second partition 212b, the first coupling protrusion 218a may be disposed at an end of a side facing the coupling groove 213b disposed in the second partition 212b among the end of the chamber cover 218. The first coupling protrusion 218a may slide inside the coupling groove 213b.

A second coupling protrusion 222a may be disposed at the other end of the chamber cover 218. A plurality of second coupling protrusions 222a may be provided. The second coupling protrusion 222a may be disposed on one surface of the first partition 212a and fixed to the first partition 212a through a coupling member (not shown) such as a bolt.

The first exposure hole 218b may be disposed at a side of the chamber cover 218 in the longitudinal direction. When the rotator 212 is rotated and the third chamber 216c faces the second connection part 150 of the housing 100, the first exposure hole 218b may connect the inside of the third chamber 216c and the second connection part 150. The first exposure hole 218b may be used as a passage through which an end of a nozzle 264 of the vaporization part 260 to be described below passes.

As shown in FIG. 5, the side cover 220 may be disposed at one side and the other side in the longitudinal direction of the rotator 212. The side cover 220 may have a shape corresponding to the shape of one side and the other side of the rotator 212 in the longitudinal direction.

The side cover 220 may include a main body 222, a first support frame 224, a second support frame 226, and a third support frame 228.

The main body 222 may have a shape in which one side is cut along a shape of the second cover frame 212f of the fourth chamber 216d. The main body 222 can be disposed at one side and the other side of the rotator 212 in the longitudinal direction to prevent the first sterilization part 230 disposed in the second chamber 216b and the second sterilization part 240 disposed in the third chamber 216c and the fourth chamber 216d from being separated. A hole through which the rotation shaft 214 may pass may be formed in the main body 222.

The main body 222 may include the second coupling protrusion 222a. The second coupling protrusion 222a may be disposed in the first chamber 216a when the side cover 220 is disposed at one side and the other side of the rotator 212 in the longitudinal direction. The second coupling protrusion 222a may come into contact with the first partition 212a and the fourth partition 212d. In a state in which the second coupling protrusion 222a comes into contact with the first partition 212a and the fourth partition 212d, the second coupling protrusion 222a may be fixed to the first partition 212a and the fourth partition 212d through the coupling member such as a bolt.

The first support frame 224 may be disposed on one surface of the main body 222. When the side cover 220 is disposed at one side and the other side of the rotator 212 in the longitudinal direction, the first support frame 224 may be disposed in the third chamber 216c. The first support frame 224 may protrude from one surface of the main body 222 in the longitudinal direction of the rotator 212. The first support frame 224 may support the chamber cover 218. Accordingly, the chamber cover 218 can prevent the first sterilization part 230 disposed in the third chamber 216c from being damaged due to the shaking of the first sterilization part 230 toward the first accommodation part 110 or the second accommodation part 120.

The first support frame 224 may include a concave portion 224a. The concave portion 224a may accommodate an end of the chamber cover 218. The end of the first partition 212a and the end of the chamber cover 218 may be interposed between the concave portion 224a and the second coupling protrusion 222a. In this state, the second coupling protrusion 222a, the first partition 212a, and the chamber cover 218 may be coupled through the coupling member such as a bolt.

The second support frame 226 may be disposed on one surface of the main body 222 on which the first support frame 224 is disposed. When the side cover 220 is disposed at one side and the other side of the rotator 212 in the longitudinal direction, the second support frame 226 may be disposed in the second chamber 216b. The second support frame 226 may be disposed to be spaced apart from the first support frame 224 with the second partition 212b interposed therebetween. The second support frame 226 may come into contact with the second partition 212b together with the first support frame 224 to stably maintain a state in which the side cover 220 is fixed to the rotator 212.

The third support frame 228 may be disposed on one surface of the main body 222 on which the first support frame 224 and the second support frame 226 are disposed. When the side cover 220 is disposed at one side and the other side of the rotator 212 in the longitudinal direction, the third support frame 228 may be disposed in the fourth chamber 216d. The fourth chamber 216d may be disposed to be spaced apart from the second support frame 226 with the third partition 212c interposed therebetween. The third support frame 228 may support the fourth chamber 216d. Although not shown, the third support frame 228 may form a guide path of the cable 420 together with the second support frame 226.

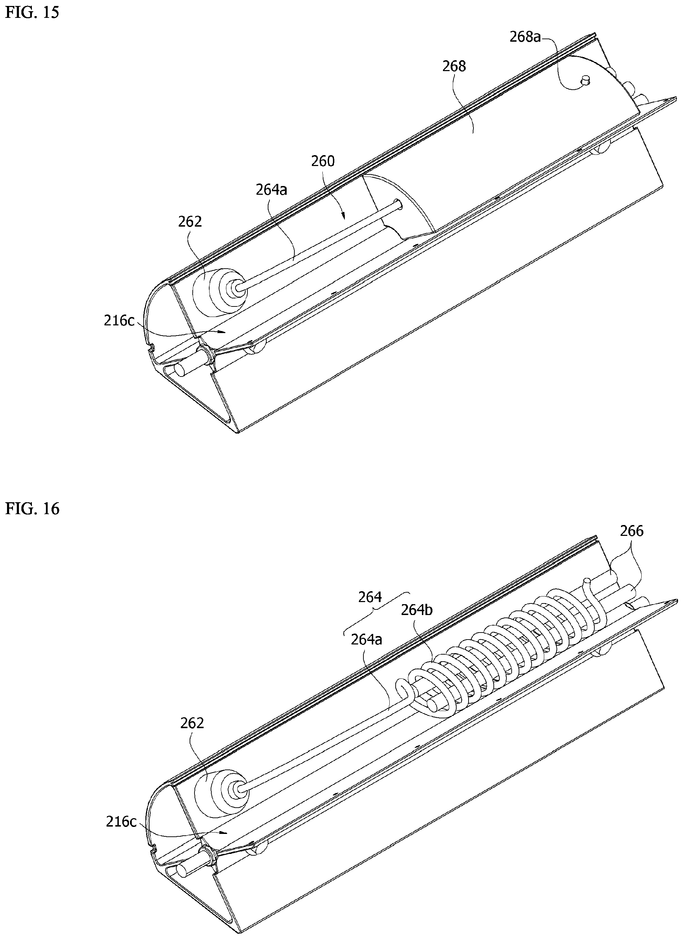

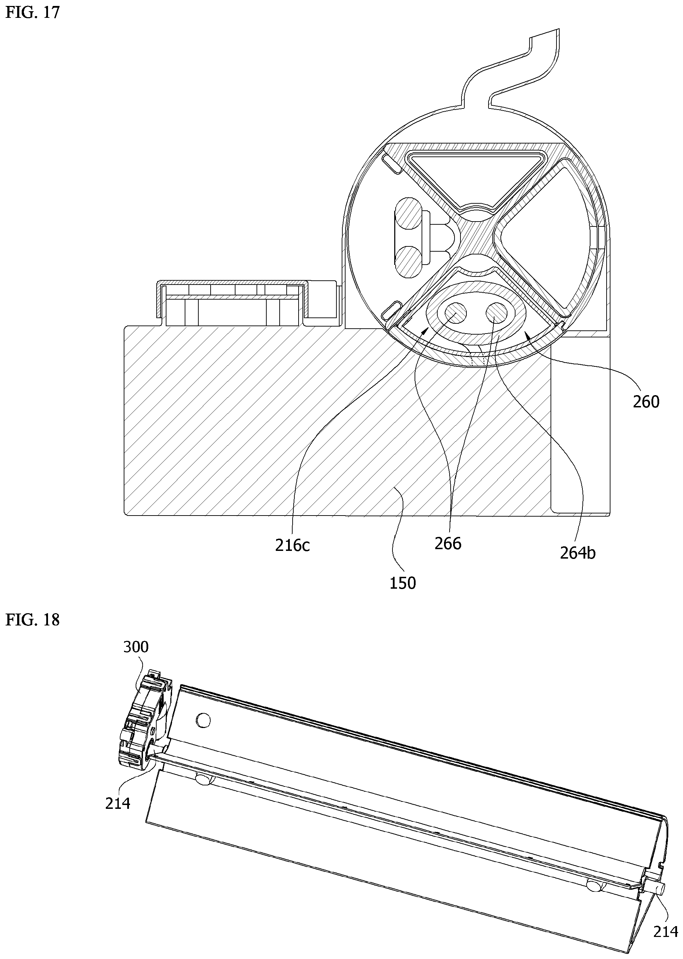

FIG. 12 is a view showing a state in which a first sterilization part is disposed in a first chamber, FIG. 13 is a view showing a state in which the first sterilization part and a second connection part are disposed to face each other, FIG. 14 is a view showing a state in which a storage part is separated from a second chamber, FIG. 15 is a view showing a state in which a vaporization part is disposed in a third chamber, FIG. 16 is a view showing a state in which a heat transfer body has been removed from the vaporization part, and FIG. 17 is a view showing a state in which the vaporization part is disposed to face the second connection part.

Referring to FIGS. 12 to 17, the first sterilization part 230 may be disposed in the first chamber 216a. When the rotator 212 is rotated, the first sterilization part 230 may be disposed to communicate with the second connection part 150 of the housing 100. Accordingly, the sterilization material generated from the first sterilization part 230 may move from the first sterilization part 230 toward the vehicle air vent through the second connection part 150 together with the air. The first sterilization part 230 may be electrically connected to the controller 400 through the cable 420. Accordingly, the first sterilization part 230 may receive a control signal generated from the controller 400 through the cable 420.

The first sterilization part 230 may generate the sterilization material. The sterilization material may contain ozone. The first sterilization part 230 may include a lamp which can generate ozone. The lamp may include a UV-C lamp using an ultraviolet-C (UV-C) LED configured to emit ultraviolet light having a wavelength range of 100 nm to 280 nm. The UV-C lamps may generate wavelengths of 185 nm and 253.7 nm that generate ozone.

The second sterilization part 240 may be disposed in each of the second chamber 216b and the third chamber 216c. The second sterilization part 240 may be configured to store the sterilization solution inside the housing 100 and vaporize the sterilization solution. When the rotator 212 is rotated, the second sterilization part 240 may be disposed to communicate with the second connection part 150 of the housing 100. Accordingly, the sterilization material generated from the second sterilization part 240 may move from the second sterilization part 240 toward the vehicle air vent through the second connection part 150 together with the air. The second sterilization part 240 may be electrically connected to the controller 400 through the cable 420. Accordingly, the second sterilization part 240 may receive a control signal generated from the controller 400 through the cable 420.

The second sterilization part 240 may include the storage part 250 and the vaporization part 260.

The storage part 250 may store the sterilization solution. The sterilization solution used in this embodiment may include a phytoncide solution. The phytoncide is a material secreted from plants to protect the plants from life such as pathogens, pests, and fungi, and mainly consists of terpene that is a polymer that chemically consists of isoprene (C5H8).

The storage part 250 may be disposed in the second chamber 216b. As shown in FIG. 14, the storage part 250 may have an outer surface shape corresponding to an inner surface shape of the second chamber 216b. Accordingly, when the storage part 250 is disposed in the second chamber 216b, damage caused by the shaking of the storage part 250 can be prevented.

The storage part 250 may include the second inflow hole 252. When the storage part 250 is disposed in the second chamber 216b, the second inflow hole 252 may be disposed at a position at which it communicates with the first inflow hole 213a. The second inflow hole 252 may communicate with the first inflow hole 213a. In addition, when the rotator 212 is rotated and the second chamber 216b is disposed at one side (e.g., the top) of the first accommodation part 110 of the housing 100, the second inflow hole 252 may communicate with the inflow pipe 160. The second inflow hole 252 may be used as the passage of the sterilization solution together with the inflow pipe 160 and the first inflow hole 213a. The sterilization solution may be accommodated inside the storage part 250 through the second inflow hole 252.

The vaporization part 260 may generate mist by vaporizing the sterilization solution stored in the storage part 250. The vaporization part 260 may be disposed in the third chamber 216c.

The vaporization part 260 may include a pump 262, the nozzle 264, a heater 266, and a heat transfer body 268.

The pump 262 may pump the sterilization solution stored in the storage part 250. More specifically, the pump 262 may be disposed in the third chamber 216c, and may pump the sterilization solution stored in the storage part 250 disposed in the second chamber 216b in a state of being disposed in the third chamber 216c. At this time, the pump 262 may receive a control signal from the controller 400 through the cable 420 to pump the sterilization solution.

The nozzle 264 is connected to the pump 262. The nozzle 264 may be connected to the pump 262 to guide the sterilization solution pumped through the pump 262. The nozzle 264 may include a first transport part 264a having a shape extending straightly from the pump 262, and a second transport part 264b disposed at an end of a straight portion and having a spring shape to form a space therein. The nozzle 264 may include a material having strong heat resistance to withstand the heat transferred through a heating part. An end of the second transport part 264b may be exposed to the outside of the chamber cover 218 by the first exposure hole 218b. Accordingly, when the rotator 212 is rotated and the third chamber 216c is disposed to face the second connection part 150 of the housing 100, the end of the second transport part 264b may communicate with the second connection part 150.

The heater 266 may be disposed in an inner space formed through the second transport part 264b of the nozzle 264. The heater 266 may be provided in a cylindrical shape formed in the longitudinal direction of the rotator 212.

The heater 266 may be wound around the nozzle 264, and may heat the sterilization solution that moves inside the nozzle 264. The heater 266 may be disposed inside the heat transfer body 268, and one end of the heater 266 may protrude to the outside of the heat transfer body 268. One end of the heater 266 may be connected to the cable 420 to receive the control signal from the controller 400 to generate heat.

The heater 266 may heat the sterilization solution that moves inside the second transport part 264b of the nozzle 264. The sterilization solution may be vaporized by heat generated from the heater 266 while moving inside the second transport part 264b of the nozzle 264.

The heat transfer body 268 may accommodate the second transport part 264b of the nozzle 264 and the heater 266 therein, and transfer the heat generated from the heater 266 to the nozzle 264. The heat transfer body 268 may have a hollow shape to accommodate the second transport part 264b of the nozzle 264 and the heater 266. The heat transfer body 268 may have an outer surface shape corresponding to an inner shape of the third chamber 216c. In addition, the heat transfer body 268 may come into contact with the inner surfaces of the first partition 212a, the second partition 212b, and the chamber cover 218. Accordingly, the heat transfer body 268 is not shaken inside the third chamber 216c, and thus the heat generated from the heater 266 can be stably transferred to the second transport part 264b of the nozzle 264.

The heat transfer body 268 may include a second exposure hole 268a. When the chamber cover 218 is disposed at one side (e.g., a top) of the heat transfer body 268, the second exposure hole 268a may be disposed at a position at which it communicates with the first exposure hole 218b. The second exposure hole 268a may allow an end of the second transport part 264b of the nozzle 264 to pass therethrough together with the first exposure hole 218b. Accordingly, the end of the second transport part 264b may be exposed to the outside of the chamber cover 218.

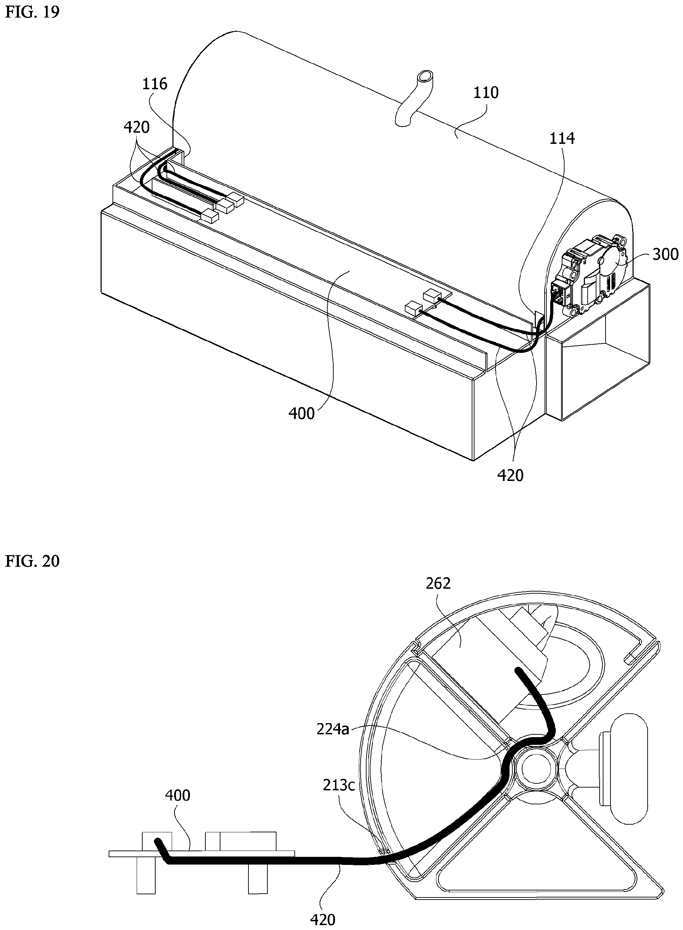

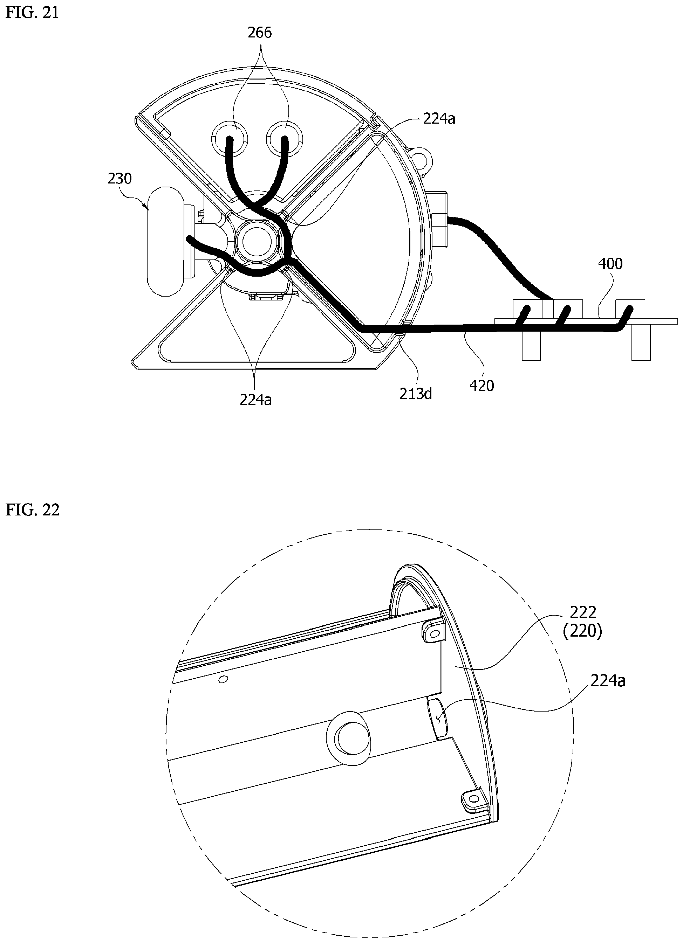

FIG. 18 is a view showing a state in which a drive source is coupled to the rotary shaft, FIG. 19 is a view showing a state in which a controller case has been removed from a housing, FIG. 20 is a view showing a cable connected to a pump and a controller, FIG. 21 is a view showing a cable connected to the first sterilization part and the controller and a cable connected to a heater and the controller, and FIG. 22 is a view showing a state in which a side cover covers an open one side of a concave portion.

Referring to FIGS. 1 and 18, the drive source 300 may be coupled to the rotation shaft 214 of the rotation part 210. More specifically, the drive source 300 may be coupled to the end of the rotation shaft 214 exposed to the outside of the housing 100. Accordingly, the drive source 300 may be disposed at the outside of the housing 100. The drive source 300 may receive the control signal from the controller 400 through the cable 420. The drive source 300 receiving the control signal may generate a driving force. The rotation shaft 214 may be rotated by the driving force generated from the drive source 300. The rotator 212 may be rotated by the rotation of the rotation shaft 214. The drive source 300 may include an actuator but is not limited thereto.

Although not shown, two drive sources 300 may be coupled to the rotation shafts 214 exposed to both sides of the housing 100. Accordingly, the rotation shaft 214 may be stably rotated, and the rotator 212 may be rotated stably.

Referring to FIG. 19, the controller 400 may be disposed inside the holding frame 170 of the housing 100. The controller 400 may include a printed circuit board (PCB).

The controller 400 may transmit a signal for rotating the rotation shaft 214 of the sterilization unit 200 to the drive source 300 so that the first sterilization part 230 and the second connection part 150 communicate with each other, and transmit a signal for rotating the rotation shaft 214 of the sterilization unit 200 to the drive source 300 so that the second sterilization part 240 and the second connection part 150 communicate with each other. When the rotation shaft 214 is rotated by the signal of the controller 400, the first sterilization part 230 or the second sterilization part 240 may be rotated while the rotator 212 connected to the rotation shaft 214 is rotated.

The controller 400 may transmit a signal for operating the first sterilization part 230 to the first sterilization part 230 in a state in which the first sterilization part 230 and the second connection part 150 communicate with each other. Accordingly, the sterilization material may be generated through the first sterilization part 230, and the sterilization material may pass through the second connection part 150 from the first sterilization part 230 to move toward the vehicle air vent.

The controller 400 may transmit a signal for operating the second sterilization part 240 to the second sterilization part 240 in a state in which the second sterilization part 240 and the second connection part 150 communicate with each other. More specifically, the controller 400 may transmit a control signal for operating the pump 262 to the pump 262 so that the pump 262 may pump the sterilization solution stored in the storage part 250. In addition, the controller 400 may transmit a control signal for operating the heater 266 to the heater 266 so that the heater 266 may generate heat.

The controller 400 may include the cable 420. A plurality of cables 420 may be provided. Each of the cables 420 may have one end connected to the controller 400, and the other end connected to each of the first sterilization part 230, the pump 262, the heater 266, and the drive source 300.

Referring to FIG. 20, the cable 420 configured to connect the controller 400 and the pump 262 may pass through the first through hole 114 and the first side groove 213c and may be drawn into the first accommodation part 110 or drawn out to the outside of the housing 100. The cable 420 introduced into the first accommodation part 110 may be guided to the concave portions 224a formed in the first partition 212a to the fourth partition 212d and aligned inside the first accommodation part 110.

Referring to FIG. 21, the cables 420 configured to connect the controller 400 and the first sterilization part 230 and connect the controller 400 and the heater 266 may pass through the second through hole 116 and the second side groove 213d and may be drawn into the first accommodation part 110 or drawn out to the outside of the housing 100. The cable 420 drawn into the second accommodation part 120 may be guided to the concave portions 224a formed in the first partition 212a to the fourth partition 212d and aligned inside the first accommodation part 110.

In this embodiment, it is exemplified that the cable 420 connected to the heater 266 is disposed at one end (e.g., an upper end) of the concave portion 224a, and the cable 420 connected to the first sterilization part 230 is disposed at the other end (e.g., a lower end) of the concave portion 224a, but the present disclosure is not limited thereto. The cable 420 connected to the first sterilization part 230 may be disposed at one end (e.g., the upper end) of the concave portion 224a, and the cable 420 connected to the heater 266 may also be disposed at the other end (e.g., the lower end) of the concave portion 224a.

Referring to FIG. 22, the open side of the concave portion 224a may be closed by the side cover 220. The cable 420 accommodated inside the concave portion 224a may be prevented from being separated to the outside of the concave portion 224a by the side cover 220. Accordingly, the cable 420 guided to the concave portion 224a may stably transmit the control signal transmitted from the controller 400 to the first sterilization part 230, the pump 262, and the heater 266.

Referring to FIGS. 1, 2, and 19, the controller case 500 may be disposed above the second accommodation part 120 of the housing 100. The controller case 500 may cover the holding frame 170. Accordingly, the controller case 500 may prevent the controller 400 disposed inside the holding frame 170 from being separated to the outside of the holding frame 170. Grooves through which the cables 420 pass may be formed at one end and the other end of the controller case 500 so that the cable 420 configured to connect the actuator and the controller 400 may be disposed inside the holding frame 170.

Hereinafter, a sterilization system for a vehicle according to one embodiment of the present disclosure will be described. When describing the sterilization system for a vehicle, the same reference numerals are used for components overlapping those of the sterilization apparatus for a vehicle according to one embodiment of the present disclosure, and a description thereof will be omitted.

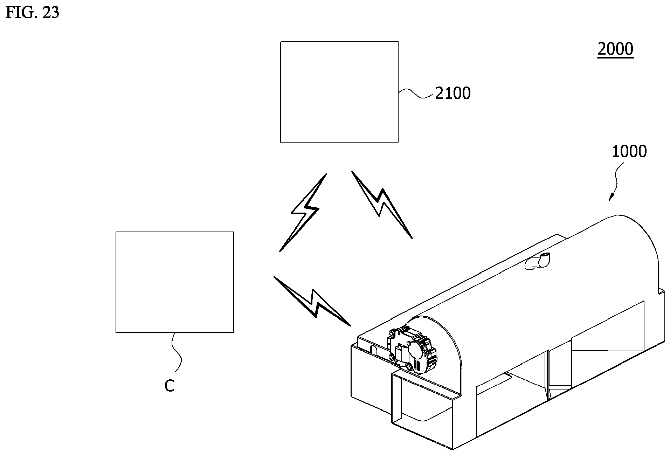

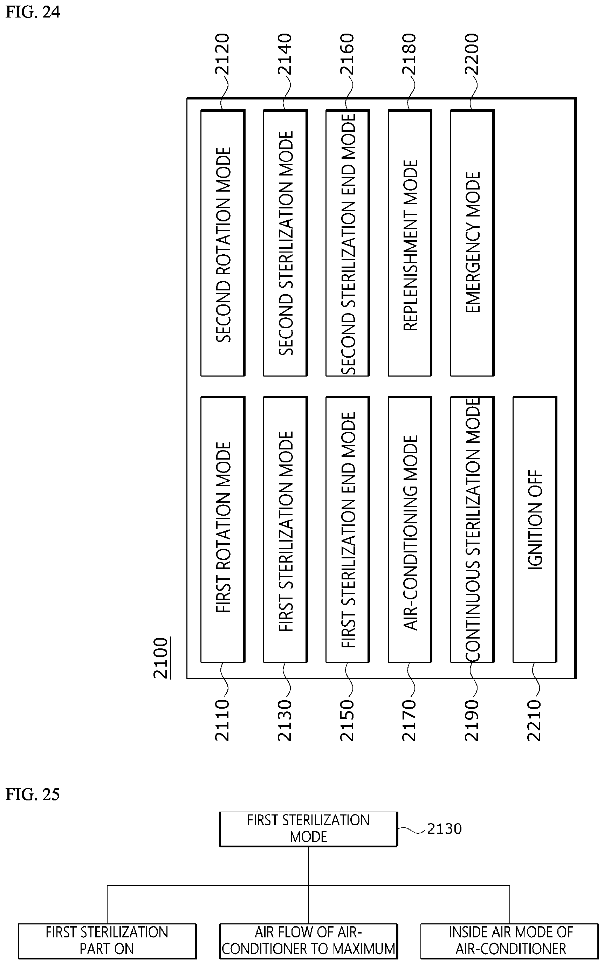

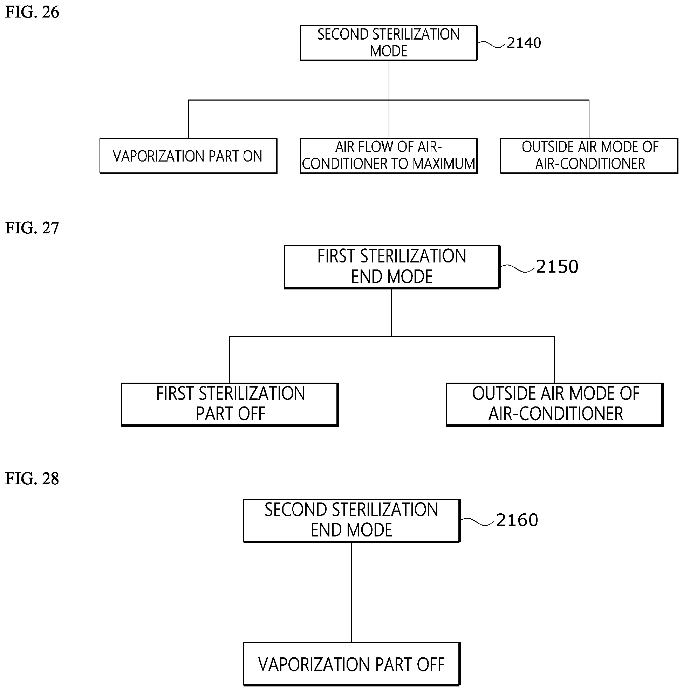

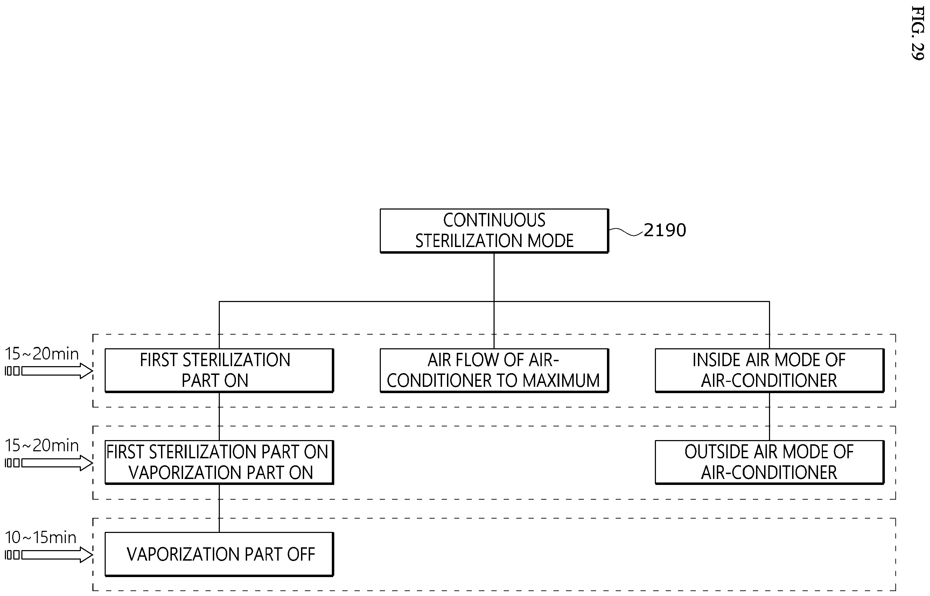

FIG. 23 is a view schematically showing a sterilization system for a vehicle according to one embodiment of the present disclosure, FIG. 24 is a view showing a configuration of a display, FIG. 25 is a view showing an operation order of a first sterilization mode, FIG. 26 is a view showing an operation order of a first sterilization end mode, FIG. 27 is a view showing an operation order of a second sterilization mode, FIG. 28 is a view showing an operation order of a second sterilization end mode, and FIG. 29 is a view showing an operation order of a continuous sterilization mode.

Referring to FIGS. 23 and 24, a sterilization system 2000 for a vehicle according to one embodiment of the present disclosure may include a sterilization apparatus 1000 for a vehicle configured to generate a sterilization material for disinfecting a vehicle interior and a display 2100 configured to control the sterilization apparatus 1000 for a vehicle.

Since the sterilization apparatus 1000 for a vehicle has the same configuration as that of the sterilization apparatus 1000 for a vehicle according to one embodiment of the present disclosure, a description thereof will be omitted.

The display 2100 may transmit a control signal to each of the controller 400 of the sterilization apparatus 1000 for a vehicle and a control unit C (e.g., processor, CPU, etc.) of the vehicle through a communication device (not shown). The display 2100 and the controller 400 of the sterilization apparatus 1000 for a vehicle or the display 2100 and the control unit C of the vehicle may transmit and receive the control signal in a wireless communication manner through the communication device.

The communication device used herein may include metropolitan area network (MAN), wide area network (WAN), Internet, third generation (3G) or fourth generation (4G) mobile communication network, Wi-Fi, wireless broadband Internet (WIBRO), and/or long term evolution (LTE), but is not limited thereto, and various wireless communications may be used.

The display 2100 may provide an interface configured to generate a control signal for transmitting the control signal to the controller 400 of the sterilization apparatus 1000 for a vehicle or the control unit C of the vehicle. To this end, the display 2100 may include at least one of a smartphone, a tablet PC, a notebook computer, and/or a PC.

The display 2100 may include a first rotation mode 2110, a second rotation mode 2120, a first sterilization mode 2130, a second sterilization mode 2140, a first sterilization end mode 2150, a second sterilization end mode 2160, an air-conditioning mode 2170, a replenishment mode 2180, a continuous sterilization mode 2190, an emergency mode 2200, and an ignition OFF mode 2210.

When the first rotation mode 2110 is selected, the display 2100 may generate a control signal for generating the driving force of the drive source 300 so that the first chamber 216a formed on the rotator 212 may face the second connection part 150 of the housing 100. The generated control signal may be transmitted from the display 2100 to the controller 400 of the vehicle through the communication device. The controller 400 may transmit the control signal for generating the driving force of the drive source 300 to the drive source 300 through the cable 420 based on the received control signal.

When the second rotation mode 2120 is selected, the display 2100 may generate a control signal for generating the driving force of the drive source 300 so that the third chamber 216c formed on the rotator 212 may face the second connection part 150 of the housing 100 to transmit the control signal to the controller 400.

Referring to FIG. 25, when the first sterilization mode 2130 is selected, the display 2100 may generate a control signal for operating the first sterilization part 230 to transmit the control signal to the controller 400. In addition, when the first sterilization mode 2130 is selected, the display 2100 may transmit a control signal for operating an air flow of the air conditioner to the maximum and a control signal for switching the air conditioner to an inside air mode to the control unit C of the vehicle.

Referring to FIG. 26, when the second sterilization mode 2140 is selected, the display 2100 may generate a control signal for operating the second sterilization part 240 to transmit the control signal to the controller 400. More specifically, when the second sterilization mode 2140 is selected, the display 2100 may generate a control signal for pumping the sterilization solution stored in the storage part 250 through the pump 262 and generate a control signal for generating heat from the heater 266. In addition, when the second sterilization mode 2140 is selected, the display 2100 may transmit the control signal for operating the air conditioner to the maximum and a control signal for switching the air conditioner to an outside air mode to the control unit C of the vehicle.

Referring to FIG. 27, when the first sterilization end mode 2150 is selected, the display 2100 may generate a control signal for stopping the operation of the first sterilization part 230 to transmit the control signal to the controller 400. In addition, the display 2100 may transmit a control signal for switching the air conditioner from the inside air mode to the outside air mode to the control unit C of the vehicle.

Referring to FIG. 28, when the second sterilization end mode 2160 is selected, the display 2100 may generate a control signal for stopping the operation of the second sterilization part 240 to transmit the control signal to the controller 400. More specifically, the display 2100 may generate a control signal for stopping the pumping of the pump 262 and a control signal for stopping the operation of the heater 266.

When the air-conditioning mode 2170 is selected, the display 2100 may generate a control signal for generating the driving force of the drive source 300 so that the fourth chamber 216d formed on the rotator 212 may face the second connection part 150 of the housing 100 to transmit the control signal to the controller 400.

When the replenishment mode 2180 is selected, the display 2100 may generate a control signal for generating the driving force of the drive source 300 so that the fourth chamber 216d formed on the rotator 212 may face the second connection part 150 of the housing 100 to transmit the control signal to the controller 400. In addition, the display 2100 may control the interface so that a user may not select modes other than the replenishment mode 2180 in the state of the replenishment mode 2180. In addition, when the replenishment of the sterilization solution is finished, the display 2100 may activate the interface so that the user may select modes other than the replenishment mode 2180.

Referring to FIG. 29, when the continuous sterilization mode 2190 is selected, the display 2100 may sequentially execute the first rotation mode 2110, the first sterilization mode 2130, the second rotation mode 2120, the second sterilization mode 2140, and the second sterilization end mode 2160. At this time, although not shown, the display 2100 may activate an interface that enables the user to set the first sterilization mode 2130 to a desired time between 15 minutes and 20 minutes, activate an interface that enables the user to set the second sterilization mode 2140 to a desired time between 15 minutes and 20 minutes, and activate an interface that enables the user to set the second sterilization end mode 2160 to a desired time between 10 minutes and 15 minutes.

When the first sterilization mode 2130 or the second sterilization mode 2140 is selected and the user urgently needs to use the vehicle while the sterilization apparatus for a vehicle is performing the sterilization operation, the user may end the first sterilization mode 2130 or the second sterilization mode 2140 through the emergency mode 2200. When the emergency mode 2200 is selected, the display 2100 may transmit a control signal for ending the first sterilization mode 2130 to the controller 400, activate an interface which can select the inside air mode or the outside air mode of the air conditioner for a vehicle, and activate an interface which can select an activation time of the inside air mode or the outside air mode. When the emergency mode 2200 is ended, the display 2100 may receive an end signal from the control unit C of the vehicle to activate an interface for notifying the user of the end.

The ignition OFF mode 2210 may transmit a control signal for stopping the starting of the vehicle to the control unit C of the vehicle.

Although not shown, the display 2100 may activate an interface for controlling the driving force of the drive source 300 so that the rotation shaft 214 of the rotation part 210 may be rotated at an arbitrary set angle. For example, the display 2100 may activate an interface for controlling the power of the drive source 300 so that the rotation shaft 214 may rotate by 90 degrees. In addition, the display 2100 may activate an interface for operating the drive source 300 so that the rotation shaft 214 is rotated in any one of a clockwise direction and a counterclockwise direction.

As described above, the sterilization system 2000 for a vehicle may remotely sterilize the passenger compartment through the display 2100 and ventilate the sterilized passenger compartment by activating the air-conditioning system for a vehicle. Accordingly, it is possible to provide an optimal condition for the passenger to feel a sense of stability in the passenger compartment through the sterilization system 2000 for a vehicle.

According to one embodiment of the present disclosure, it is possible to improve the cleanliness inside a passenger compartment by performing a strong sterilization operation inside the passenger compartment.

In addition, according to one embodiment of the present disclosure, it is possible to maintain design quality of an external appearance of a cockpit package because the sterilization apparatus is installed inside the cockpit package for a vehicle.

Although the above has been described with reference to the embodiments of the present disclosure, those skilled in the art will be able to understand that the present disclosure may be variously modified and changed without departing from the spirit and scope of the present disclosure described in the appended claims. In addition, differences related to these modifications and changes should be construed as being included in the scope of the present disclosure defined in the appended claims.

## Claims

1. A sterilization apparatus (1000) for a vehicle, comprising:
a housing (100) including a connection part (140, 150) connected to a vent of the vehicle;
a sterilization unit (200) disposed inside the housing (100), and configured to generate a sterilization material and to discharge the sterilization material toward the vent; and
a drive source (300) configured to drive the sterilization unit (200) so that the sterilization unit (200) communicates with the connection part (140, 150) to enable the sterilization material to be discharged.

2. The sterilization apparatus of claim 1, wherein the sterilization unit (200) includes:
a first sterilization part (230) configured to generate ozone; and
a second sterilization part (240) configured to store a sterilization solution inside the housing (100) and to vaporize the sterilization solution.

3. The sterilization apparatus of claim 2, wherein the sterilization unit (200) includes a rotation part (210) configured to rotate the first sterilization part (230) and the second sterilization part (240), and
the rotation part (210) includes: a rotator (212) including a plurality of chambers (216) in which the first sterilization part (230) and the second sterilization part (240) are disposed; and a rotation shaft (214) connected to the rotator (212) and the drive source (300) to rotate the rotator (212) through power generated from the drive source (300).

4. The sterilization apparatus of claim 3, wherein when the rotator (212) rotates, the first sterilization part (230) and the second sterilization part (240) selectively communicate with the connection part (140, 150) of the housing (100).

5. The sterilization apparatus of claim 3 or 4, wherein the second sterilization part (240) includes:
a storage part (250) configured to store the sterilization solution; and
a vaporization part (260) configured to generate mist by vaporizing the sterilization solution stored in the storage part (250).

6. The sterilization apparatus of claim 5, wherein the plurality of chambers (216) include:
a first chamber (216a) in which the first sterilization part (230) is disposed;
a second chamber (216b) in which the storage part (250) is disposed;
a third chamber (216c) in which the vaporization part (260) is disposed; and
a fourth chamber (216d) having a closed shape so as not to communicate with the connection part (140, 150) of the housing (100) in an air-conditioning operating state of an air-conditioning apparatus.

7. The sterilization apparatus of claim 6, wherein the vaporization part (260) includes:
a pump (262) configured to pump the sterilization solution stored in the storage part (250);
a nozzle (264) connected to the pump (262) to guide the pumped sterilization solution;
a heater (266) wound around the nozzle (264), and configured to heat the sterilization solution that moves inside the nozzle (264); and
a heat transfer body (268) configured to accommodate a part of the nozzle (264) and the heater (266) therein and to transmit heat generated from the heater (266) to the nozzle (264).

8. The sterilization apparatus of claim 7, wherein the sterilization unit (200) includes a chamber cover (218) configured to cover an open one side of the third chamber (216c),
the chamber cover (218) includes a first exposure hole (218b) through which an end of the nozzle (264) passes, and
the heat transfer body (268) includes a second exposure hole (268a) configured to communicate with the first exposure hole (218b), wherein the end of the nozzle (264) passes through the second exposure hole (268a) together with the first exposure hole (218b).

9. The sterilization apparatus of claim 8, wherein the rotator (212) includes a first inflow hole (213a) formed in an area corresponding to the second chamber (216b),
the storage part (250) includes a second inflow hole (252) communicating with the first inflow hole (213a), and
the housing (100) includes an inflow pipe (160) configured to communicate with the first inflow hole (213a) and the second inflow hole (252), and to guide the sterilization solution to an inside of the storage part (250).

10. The sterilization apparatus of any one of claims 1 to 9, a controller (400) electrically connected to the drive source (300) to transmit a rotation signal to the drive source (300),
wherein the sterilization unit (200) is rotated by the drive source (300) to form a movement path of the sterilization material together with the connection part (140, 150) and the vent.
